# EUROPEAN PATENT APPLICATION

(11) **EP 1 034 782 A1**
(43) Date of publication of application: **13.09.2000**
(21) Application number: 99104359.7
(22) Date of filing: 04.03.1999
(51) Int. Cl.: A61K 31/12, A61K 35/78, G01N 33/52

(54) **Treatment of depression by using compounds which elevate the intracellular sodium concentration**

(71) Applicant: Singer, Andrea, 70839 Gerlingen (DE); Wonnemann, Meinolf, 60385 Frankfurt (Main) (DE); Müller, Walter E., Prof.Dr., 67550 Worms (DE)
(72) Inventor: Singer, Andrea, 70839 Gerlingen (DE); Wonnemann, Meinolf, 60385 Frankfurt/Main (DE)

(57) **Abstract**

Hyperforin is the major lipophilic constituent of the medicinal plant St. John's wort (Hypericum perforatum L.). Extracts of Hypericum perforatum are broadly used for the treatment of depressive disorders and are unspecific inhibitors of the neuronal uptake of several neurotransmitters. Previous studies have shown that hyperforin represents the reuptake inhibiting constituent.

To characterize the mechanism of serotonin reuptake inhibition, kinetik analyses in synaptosomes of mouse brain were performed. Michaelis-Menten kinetics revealed that hyperforin (2µM) induced a decrease in Vₘₐₓ from 0,191 ± 0,034 pM/min/mg protein to 0,083 ± 0,032 pM/min/mg protein without effecting Kₘ, indicating non-competitive inhibiting properties of the substance. Similar effects were seen on the uptake systems of GABA and L-glutamate. By contrast, citalopram (1 nM), a competitive inhibitor of serotonin uptake, leads to an elevation of Kₘ without changing Vₘₐₓ. Monensin a Na⁺/H⁺-exchanger substance showed the same properties (Vₘₐₓ: 0,065 ± 0,009 pM/min/mg protein, Kₘ: 14,72 ± 3,94 nM) as hyperforin. To further compare the mechanism of action of hyperforin to classical antidepressants like SSRI's and TCA's, the inhibition of ³H-paroxetine binding (a selective marker of the serotonin transporter) was studied. The correlation between IC₅₀-values for specific ³H-paroxetine binding and for ³H-serotonin uptake inhibition was significant for many antidepressants but not for hyperforin or monensin. As monensin works by elevating free intracellular Na⁺ we compared the effects of hyperforin and monensin on [Na⁺]ᵢ-concentration in platelets by measuring SBFI fluorescence. Both drugs elevated [Na⁺]ᵢ from basal levels up to 69,0 ± 16,1 mM (50 µM hyperforin) and 140,0 ± 9,1 mM (10 µM monensin) in the highest concentration investigated, while the SSRI citalopram at concentrations relevant for ³H-serotonin uptake inhibition had no effect at all on [Na⁺]ᵢ. Although the mode of action of hyperforin seems to be associated with elevated free intracellular sodium similar to monensin, the biochemical mechansim might be different, since maximal elevation of [Na⁺]ᵢ remained much below the extracellular concentration. Elevating [Na⁺]ᵢ and resulting uptake inhibition of the biogene amines and amino acid transmitters or any other neurotransmitter could mean a new kind of method for the treatment of depressive disorders or other psychiatric disorders.

## Description

### Summary

Hyperforin is the major lipophilic constituent of the medicinal plant St. John's wort (Hypericum perforatum L.). Extracts of Hypericum perforatum are broadly used for the treatment of depressive disorders and are unspecific inhibitors of the neuronal uptake of several neurotransmitters. Previous studies have shown that hyperforin represents the reuptake inhibiting constituent.

To characterize the mechanism of serotonin reuptake inhibition, kinetik analyses in synaptosomes of mouse brain were performed. Michaelis-Menten kinetics revealed that hyperforin (2µM) induced a decrease in Vₘₐₓ from 0,191 ± 0,034 pM/min/mg protein to 0,083 ± 0,032 pM/min/mg protein without effecting Kₘ, indicating non-competitive inhibiting properties of the substance. Similar effects were seen on the uptake systems of GABA and L-glutamate. By contrast, citalopram (1 nM), a competitive inhibitor of serotonin uptake, leads to an elevation of Kₘ without changing Vₘₐₓ. Monensin a Na⁺/H⁺-exchanger substance showed the same properties (Vₘₐₓ: 0,065 ± 0,009 pM/min/mg protein, Kₘ: 14,72 ± 3,94 nM) as hyperforin. To further compare the mechanism of action of hyperforin to classical antidepressants like SSRI's and TCA's, the inhibition of ³H-paroxetine binding (a selective marker of the serotonin transporter) was studied. The correlation between IC₅₀-values for specific ³H-paroxetine binding and for ³H-serotonin uptake inhibition was significant for many antidepressants but not for hyperforin or monensin. As monensin works by elevating free intracellular Na⁺ we compared the effects of hyperforin and monensin on [Na⁺]ᵢ-concentration in platelets by measuring SBFI fluorescence. Both drugs elevated [Na⁺]ᵢ from basal levels up to 69,0 ± 16,1 mM (50 µM hyperforin) and 140,0 ± 9,1 mM (10 µM monensin) in the highest concentration investigated, while the SSRI citalopram at concentrations relevant for ³H-serotonin uptake inhibition had no effect at all on [Na⁺]ᵢ. Although the mode of action of hyperforin seems to be associated with elevated free intracellular sodium similar to monensin, the biochemical mechansim might be different, since maximal elevation of [Na⁺]ᵢ remained much below the extracellular concentration. Elevating [Na⁺]ᵢ and resulting uptake inhibition of the biogene amines and amino acid transmitters or any other neurotransmitter could mean a new kind of method for the treatment of depressive disorders or other psychiatric disorders.

### Introduction

Extracts of the medicinal plant hypericum perforatum (St. John's Wort) are broadly used in many countries to treat depressive disorders. Several recent reviews of controlled clinical studies with hypericum extract come to the conclusion, that it represents an effective antidepressant principle superior to placebo (Volz, 1997; Linde et al., 1996; Wheatley, 1998; Wong et al., 1998). A favourable side-effect profile is considered as the most relevant advantage. In agreement with the possible therapeutic potential, many recent pharmacological studies with hypericum extract also support it's antidepressant activity. The extract inhibits the synaptosomal uptake of norepinephrine, serotonin and dopamine, induces β-receptor down-regulation when given subchronically to rats and is active in a large variety of behavioral models indicative of antidepressant activity (Chatterjee et al., 1998; Rolli et al., 1997; Bhattacharya et al., 1998; Butterweck et al., 1997). However, it's MAO-A and MAO-B inhibiting properties are probably too weak to significantly contribute to its antidepressant activity (Cott, 14997; Müller et al., 1997).

The extract contains a large number of constituents. If one or more is responsible for the antidepressant properties is not yet finally known. The original assumption (Suzuki et al., 1984), that the naphthodianthrone derivative hypericin is specifically relevant by inhibiting MAO-A, has not been confirmed by several studies (Cott, 14997; Müller et al., 1997). Recently, the phloroglucinol derivative hyperforin became of increasing interest as it represents the major reuptake inhibiting component of hypericum extract (Chatterjee et al., 1998; Müller et al., 1998). Moreover, it leads to elevated extracellular concentrations of serotonin, norepinephrine and dopamine after administration to rats (Kaehler et al., 1999), down-regulates β-receptors (Müller et al., 1997), is active in several behavioral models indicative of antidepressant activity (Chatterjee et al., 1998; Bhattacharya et al., 1998), leads to specific changes of the rat and human EEG typical for SSRI's (Dimpfel et al., 1998; Schellenberger et al., 1998), reaches plasma levels needed to inhibit synaptosomal uptake of several neurotransmitters at therapeutic doses of the hypericum extract (Biber et al., 1998) and even seems to be relevant for the antidepressant activity of hypericum extract in man (Laakmann et al., 1998). Thus, hyperforin has to be considered an important antidepressant constituent of hypericum extract.

Contrary to all other antidepressant drugs known, hyperforin not only potently inhibits the synaptosomal uptake of norepinephrine, dopamine and serotonin but also the uptake of the two amino acid transmitters GABA and L-glutamate (Chatterjee et al., 1998). This might point to a different mechanism of action, probably not associated with specific binding sites at the transporter molecules. Accordingly, we have performed several additional experiments comparing the molecular basis of the serotonin uptake inhibition by hyperforin with that of SSRI's like citalopram.

It was therefore an object of this invention to develop a method of screening for drugs for the treatment of depressive disorders or other psychiatric disorders. In addition it was an object to develop a method of treating depression or other psychiatric disorders.

It has been surprisingly found that this represents:
- A method of treating humans to care or prevent depression or other psychiatric disorders which comprisis treating human with an effective amount of a compound which is effective in elevating the intracellular concentration of Na⁺.
- A method for screening for a pharmaceutical compound or composition for the curative or prophylactic treatment of depression or other psychiatric disorders comprising increasing the ability of a compound or a composition for elevation the intracellular concentration of Na⁺.
- A method of using a compound or composition effective in elevating the intracellular concentration of Na⁺ for the manufacture of a medicament for the curative or prophylactic treatment of depression or other psychiatric disorders.
- A method, whereas the ability of elevating (increasing) the intracellular concentration of Na⁺ is screened in synaptosomal preparation and whereas the concentration of Na⁺ is measured with fluorescence spectroscopy and whereas dyes for Na⁺ are used.
- A method, whereas the compound or composition does not include hyperforin.
- A method, whereas the free intracellular concentration of Na⁺ is increased by the compound or composition by 30 to 120 mM for minimum above the level of the untreated cell.
- A method, whereas the compound or composition decreases the uptake of serotonin, norepinephrine, dopamine, GABA and/or L-glutamate.
- A method, whereas the compound or composition increases the uptake of Na⁺ in cells by Na⁺ -channels, Na⁺ -antiporters, Na⁺ -pumps or other Na⁺ -exchange systems.
- A method, whereas the compound or composition interacts noncompetitive with the transporters of serotonin, norepinephrine, dopamine, GABA and/or L-glutamate.
- The use of hyperforin for the manufacture of a medicament for the curative or prophylactive treatment of interactions with protozoes.
- A method, whereas the dose (of the compound or composition) - response (the intracellular concentration of Na⁺ )- curve shows a maximum.

### Methods

### Synaptosomal uptake of serotonin

Synaptosomal preparations from frontal cortex of female NMRI mice were used for serotonin uptake. The tissue was homogenized in ice cold sucrose solution (0.32 M) and diluted with 10 ml of the homogenizing medium. The nuclear fraction was eliminated by centrifugation at 750 x g for 10 min (Beckman Centrifuge, Model J2-21). The supernatant was centrifugated at 17400 x g for 20 min to obtain the crude synaptosomal pellets. This pellets were suspended in 11 ml ice cold Krebs-HEPES-buffer (150 mM NaCI, 10 mM HEPES, 6.2 mM KCI, 1.2 mM Na₂HPO₄, 1.2 mM MgSO₄, 10 mM Glucose, 10 µM Pargylin, 0.1% ascorbic acid; pH 7.4 at 37°C), aliquoted in microtiter plates, incubated in the presence of varying concentrations of the drugs tested at 37°C for 15 min in a shaking water bath and cooled on ice. The ³H-labeled tracer ( 2.9 nM serotonin) was added and the uptake started by incubation at 37°C for 4 min for which time uptake is linearly depending on time. The probes were cooled, immediately filtered through Whatman GF/B glass fiber filters and washed three times with ice cold buffer solution with a Brandel cell harvester. Filters were placed in plastic scintillation vials containing 4 ml Lumasafe scintillation cocktail (Packard). Radioactivity was measured after 12 hours. Nonspecific uptake was determined in parallel probes maintained throughout on ice or containing unlabelled serotonin (1mM serotonin).

### Kinetic analyses

The transport of ³H-serotonin into synaptosomal preparations of the frontal cortex of mice was measured by the same method mentioned above in the presence of varying concentrations of ³H-serotonin (1-30 nM). Kinetic constants for uptake were obtained by direct weighted non-linear regression of active uptake against ³H-serotonin concentration with GraphPad PRISM™.

### ³H-serotonin uptake in platelets

Blood from normal unmedicated volunteers was anticoagulated in S-Monovettes with 1.0 ml citrate solution and centrifuged for 15 min at 250 x g at room temperature. The upper two-thirds of the platelet rich plasma (PRP) were collected in a Falcon tube and centrifuged at 800 x g for 30 min at RT. The resulting pellet was resuspended in a 4-fold amount of Krebs-Henseleit phosphate buffer (KHP buffer: 6.92 g NaCl, 0.35 g KCI, 0.29 g MgSO₄, 0.16 g KH₂PO₄, 2.1 g glucose and 1.0 g ascorbic acid per I; pH 7.4 at 37°C). Platelets were preincubated in a shaking water bath for 15 min with the various agents prior to the addition of ³H-serotonin (2.9 nM). The incubation was continued to 2 min after the addition of ³H-serotonin. After this time the uptake was terminated by diluting the sampels with ice cold buffer and rapid filtration through Whatman GF/C glass fiber filters. The microtiter plates and the filters were washed 3 times with a Brandel cell harvester. The filters were dried and radioactivity was determined by liquid scintillation counting. Each point was determined in triplicate. Non-specific uptake was determined in parallel experiments containing 1mM cold serotonin.

### ³H-paroxetine binding assay

Female NMRI mice were sacrified by decapitation. Frontal cortex was homogenized in 40 volumes of TRIS buffer (50 mM TRIS HCl, 120 mM NaCl, 5 mM KCI; pH 7.4 at 0°C) in a glass Potter-Elvehjem homogenizer with teflon pestle. The homogenate was centrifuged for 10 min at 35000 x g at 4°C. The resulting pellet was resuspended in 40 volumes of buffer and recentrifuged. The pellet was resuspended in TRIS buffer at a protein concentration of 70 µg/ml. Aliquots of membrane suspension were incubated with ³H-paroxetine (0.2 nM) at room temperature for 60 minutes. Incubation was terminated by rapid filtration through Whatman GF/B glass fiber filters with a Brandel Cell Harvester. Filters were routinely pretreated with 0.025 % Brij 35. The filters were washed 7 times with ice cold buffer, dried and the radioactivity was measured by liquid scintillation spectrometry. Specific binding of ³H-paroxetine was defined as the difference between the total binding and that remaining in the presence of 10 µM fluoxetine.

### Measuring [Na⁺]ᵢ with SBFI/AM (sodium binding benzofuran isophthalate acetoxymethyl ester)

Solutions: The Na⁺-HEPES buffer consisted 10 mM HEPES, 1 mM MgSO₄, 5 mM KH₂PO₄, 5 mM KCI and 5 mM Glucose (4). The pH of this buffer was adjusted to 6.5 with NH3 (for storage of platelets) and to 7.4 for the measurement of fluorescence. The K⁺ solution was identical except for complete replacement of sodium by potassium. By mixing this solutions together any sodium concentration between 0 and 145 mM can be obtained.

Preparation of platelets and loading with SBFI/AM: Venous blood (10 ml) was anticoagulated by mixing it with 1.0 ml citrate solution in a S-Monovette. The anticoagulated blood was centrifuged at 250 x g for 15 min at room temperature.The upper two-thirds of the supernatant were gently removed with a plastic pasteur pipette. Platelet pellets were obtained from PRP by centifugation at 480 x g for 30 min at room temperature. The pellet was resuspended into Sodium-HEPES buffer, pH 6.5 at a final concentration of 3-4 x 10⁸ cells/ml. Immediately before addition to the platelets, 1 volume of SBFI/AM solution was mixed with 2 volumes of the nonionic detregent Pluronic F-127. Appropriate amounts of this solution were added to the platelets to yield a final concentration of SBFI/AM of 10 µM and platelets were incubated with the dye for 60 min at 37°C in a shaking water bath. Thereafter, platelets were again centrifuged at 480 x g for 30 min at RT. The supernatant was discarded and the platelets were taken up into sodium-HEPES buffer, pH 6.5 at a concentration of 3-4 x 10⁹ cells/ml. The platelet suspension was then stored at RT until used for the fluorescence measurements.

Measurement of SBFI/AM Fluorescence: Aliquots of platelet suspension (4 µl) were added to 950 µl of the appropriate buffer. Fluorescence was measured in quartz cuvettes using a SLM-Aminco luminescence spectrometer before and after addition of the tested drugs. Sampels were excited alternately at 345 and 385 nm (bandwidth = 5 nm) and fluorescence was recorded at 490 nm (bandwidth = 16 nm). Fluorescence excitation ratios 345/385 nm were calculated. All measurements were performed without stirring at room temperature.

Calibration of SBFI/AM fluorescence in terms of [Na⁺]ᵢ was performed according to the method of Harootunian et al. (1989). By addition of platelets to solutions of known extracellular sodium concentrations, which were made by mixing different amounts of Na⁺-HEPES and K⁺-HEPES buffer, the 345/385 nm intensity ratio was determined before and 5 minutes after addition of the ionophore gramicidin D (2 µM final concentration). Baseline [Na⁺]ᵢ (mean of 15 experiments ± SD) was about 32 mM ± 6, which is in agreement with the data by Borin and Siffert (1990).

### Determination of protein concentration

Protein concentration was determined according to the method of Bradford (1976) with the Bio-Rad Proteinassay-Kit.

### Materials

Standard laboratory chemicals were obtained from Sigma-Aldrich Chemical (Deisenhofen, Germany) or were a gift from Merck (Darmstadt, Germany). SBFI/AM and gramicidin D were purchased from Molecular Probes (Eugene, OR, USA). ³H-serotonin and ³H-paroxetine were obtained from NEN Life Science Products (Boston, MA, USA) and Pluronic F-127 and monensin from Calbiochem (Frankfurt, Germany). Citalopram was a gift from Lundbeck (Hamburg, Germany). Hyperforin was generously supplied by Schwabe (Karlsruhe, Germany). Fluoxetine was purchased from Tocris (Bristol, UK).

### Results

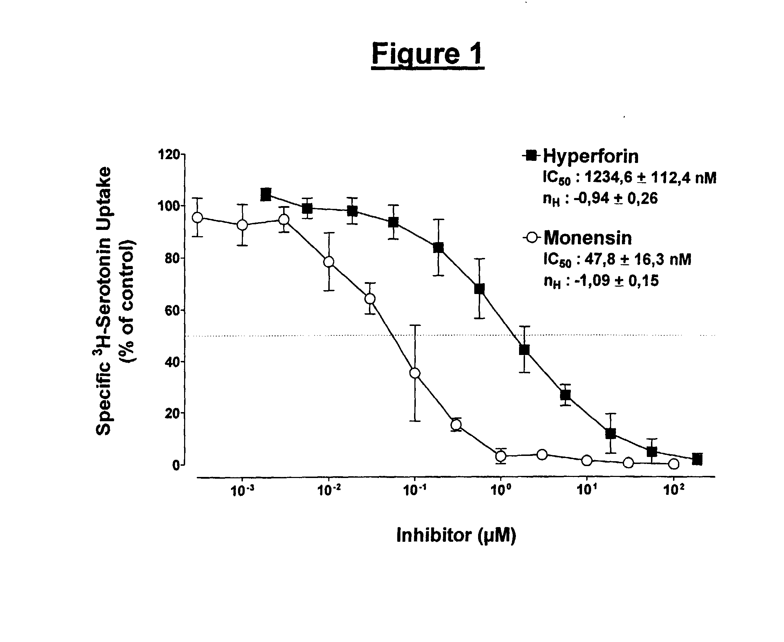

Figure 1 (Inhibition of 3H-serotonin uptake into mouse cortex synaptosomes by hyperforin and monensin. All data are means + SD of six determinations) shows the concentration dependent inhibition by hyperforin of ³H-serotonin uptake into mouse brain synaptosomes. A monophasic effect with a Hill-coefficient of about 1 was found, which is in agreement with previous findings (Müller et al., 1998, Chatterjee et al., 1998).

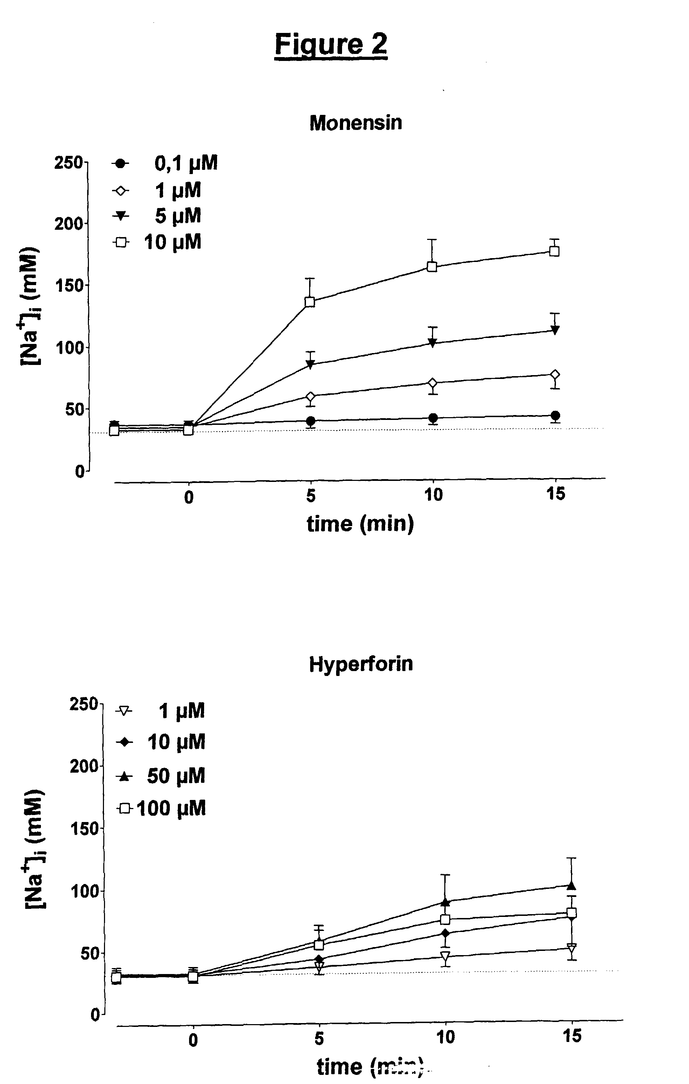

Binding of 3H-paroxetine to brain membranes labels the serotonin binding site of the human and rodent serotonin transporter molecule (Gurevich and Joyce, 1996; Marcusson and Ross, 1990). This is also supported by our findings about a good correlation between the IC₅₀-values for inhibition of ³H-paroxetine binding and for inhibition of ³H-serotonin uptake (Figure 2 :The relationship between the IC₅₀-values of several antidepressant drugs for inhibiting ³H-serotonin uptake into mouse cortex synaptosomes or inhibition of ³H-paroxetine binding to mouse cortex membranes (see also data of table 1)). For the antidepressant drugs investigated ratios varied between 1 and 3 (Table 1). However, the inhibitory effect of hyperforin on ³H-serotonin uptake is much stronger than it's potency to inhibit ³H-paroxetine binding (ratio = 15) (Table 1).

**TABLE 1**

| **Half-maximal inhibitory concentrations (IC**_{**50**}**) for specific** ^{**3**}**H-serotonin uptake and for specific** ^{**3**}**H-paroxetine binding. Data are means ± SD of six experiments** | | | |
|---|---|---|---|
| **IC**_{**50**} **(nM)** | | | |
| **Substance** | ^{**3**}**H-Paroxetine binding** | ^{**3**}**H-serotonin uptake** | **Ratio*** |
| Clomipramine | 1,9 ± 0,4 | 1,0 ± 0,1 | 1,9 |
| Sertraline | 3,3 ± 0,3 | 2,1 ± 1,0 | 1,6 |
| Citalopram | 4,1 ± 0,6 | 1,1 ± 0,2 | 3,7 |
| Fluvoxamine | 13,5 ± 3,7 | 10,9 ± 5,6 | 1,2 |
| Fluoxetine | 26,9 ± 0,6 | 10,4 ± 5,1 | 2,6 |
| Monensin | > 1000 | 47,8 ± 16,3 | ---- |
| Desipramine | 672,3 ± 171,6 | 214,3 ± 89,5 | 3,1 |
| Hyperforin | 19528 ± 6983 | 1357± 171 | 14,4 |

| | | | |
|---|---|---|---|
| *is IC₅₀ ³H-Paroxetine binding/IC₅₀ ³H-serotonin uptake | | | |

Our kinetic analyses (Table 2) indicate that hyperforin (2 µM) decreases Vₘₐₓ of synaptosomal serotonin uptake from 0.191 ± 0.034 to 0.083 ± 0.032 pM/min/mg protein. By contrast, hyperforin has no effect on Kₘ (Table 2), suggesting non-competitive inhibition by this substance. Similar effects were seen on the uptake systems of GABA and L-glutamate (data not shown). In comparision, the SSRI citalopram (1 nM) leads to an increase in Kₘ without affecting the Vₘₐₓ-value, confirming previous findings indicative of competitive inhibition of ³H-serotonin uptake (Hyttel, 1978). Both findings do not support the assumption of a competitive binding close to the serotonin binding site of the transporter molecule as it is the case with most antidepressant drugs. Therefore, we investigate other parameters known to affect ³H-seroton in uptake.

It is generally accepted that serotonin uptake requires external sodium (Sneddon, 1996; Bogdanski et al., 1979; Sneddon, 1973) and can be inhibited by the sodium ionophore monensin (Reith and O'Reilly, 1990; Izenwasser et al., 1992). The potent inhibition of synaptosomal uptake by monensin could be confirmed (Table 1). Moreover, monensin also represents a non-competitive inhibitor (Table 2) and has no affinity fnr the transporter binding site labeled with ³H-paroxetine (Table 1).

**TABLE 2**

| **Kinetic analyses of the effects of citalopram, hyperforin and monensin on** ^{**3**}**H-serotonin uptake into mouse cortex synaptosomes. Data are means ± SD of six determinations.** | | |
|---|---|---|
| | **K**_{**m**} **(nM)** | **V**_{**max**} **(pM/min/mg)** |
| **Control** | 12,72 ± 5,01 | 0,105 ± 0,044 |
| **Citalopram (1 nM)** | 29,24 ± 5,25*** | 0,106 ± 0,074 |
| **Control** | 11,90 ± 2,07 | 0,191 ± 0,034 |
| **Hyperforin (2 µM)** | 9,93 ± 0,59* | 0,083 ± 0,032*** |
| **Control** | 13,78 ± 2,50 | 0,133 ± 0,030 |
| **Monensin (100 nM)** | 14,72 ± 3,94 | 0,065 ± 0,009*** |

| | | |
|---|---|---|
| *** indicate p < 0,05 and** | | |
| ***** indicate p < 0,001 using unpaired t test.** | | |

To investigate the possible relevance of the sodium gradient for the uptake inhibition, we examined the effect of monensin on the free intracellular Na⁺ concentration using the Na⁺ indicator SBFI/AM. As this method requires incubation at 37°C for an extended period, the experiments were performed with human platelets. Platelets have repeatedly been used as a model system for the serotonin transport into neurons (Sneddon, 1973). Comparision of 3H-serotonin uptake inhibition in mouse brain synaptosomes and human platelets shows comparable IC₅₀-values for hyperforin, monensin and several antidepressants drugs in both systems (Table 3).

**TABLE 3**

| **IC**_{**50**}**-values for inhibition of** ^{**3**}**H-serotonin uptake into mouse cortex synaptosomes or human platelets. Data are means ± SD of six determinations** | | |
|---|---|---|
| | **IC**_{**50**} **(nM)** | |
| **Substance** | **Synaptosomes** | **Platelets** |
| Citalopram | 1,1 ± 0,2 | 1,2 ± 0,5 |
| Clomipramin | 1,0 ± 0,1 | 0,2 ± 0,1 |
| Hyperforin | 1234,6 ± 112,4 | 887,0 ± 153,9 |
| Monensin | 47,8 ± 16,3 | 159,5 ± 40,6 |

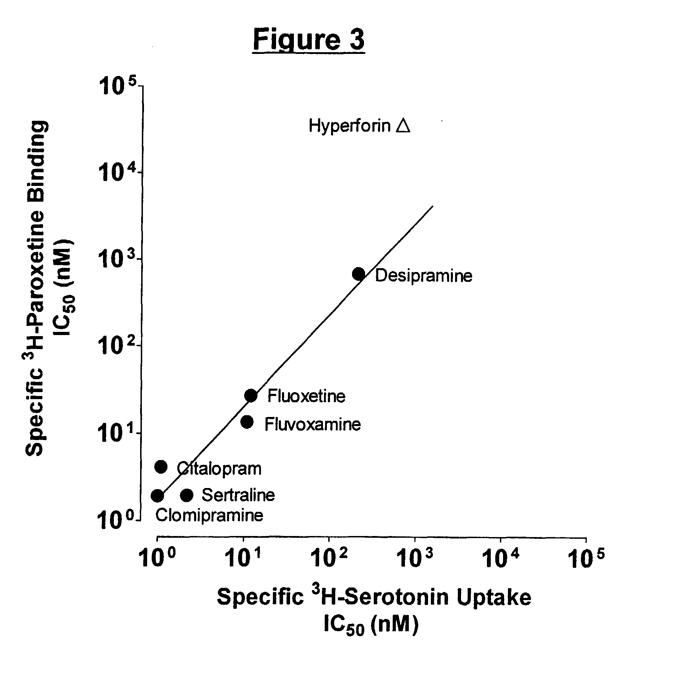

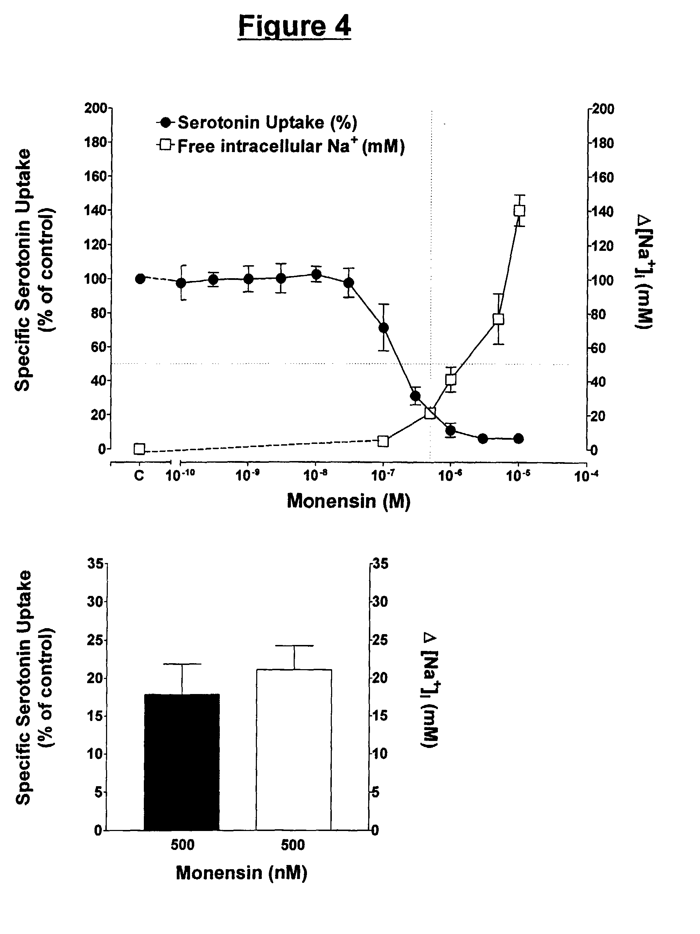

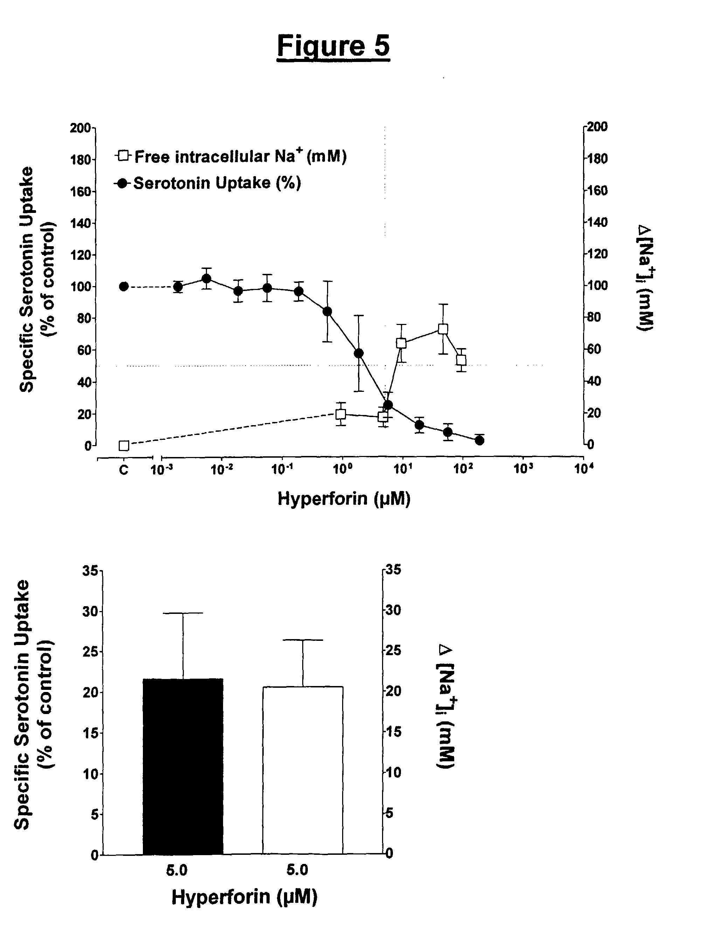

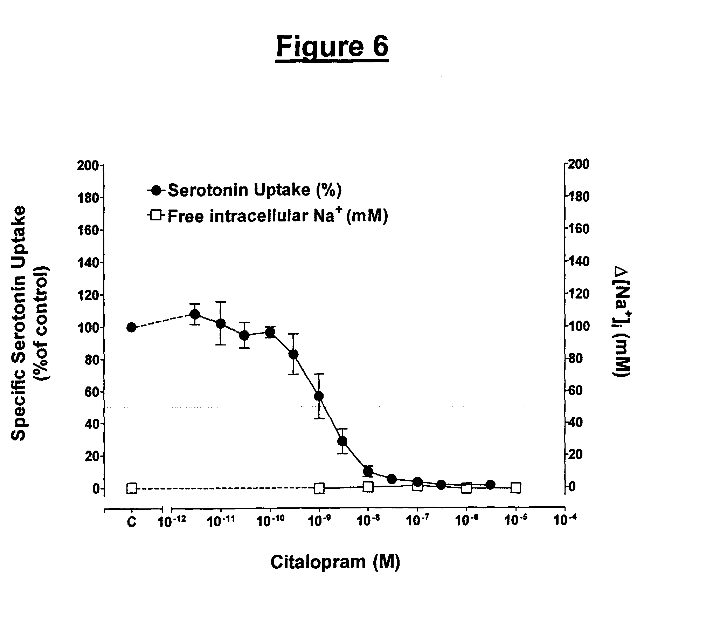

The sodium ionophore monensin has been shown to induce sodium-proton exchange across cellular membrane (Riddell et al., 1988; Katsuyoshi and Yoshihiko, 1991; Sandeaux et al., 1982). This could be confirmed for human platelets, where monensin elevated intracellular Na⁺ concentrations in a time and concentration depending fashion (Figure 3: Time courses of the effect of monensin and hyperforin on [Na+]i in human platelets. Data are means + SD of six determinations)Figure 6: Effects of increasing concentrations of citalopram on 3H-serotonin uptake and [Na+]i in human platelets. Data are means + SD of six determinations). Equilibration was obtained after 15 minutes incubation. Maximal effects were seen in the presence of high concentrations of monensin leading to an elevation of [Na+]i to extracellular level (Fig. 6). There was a close relationship between the effects of monensin on [Na+]i and on serotonin uptake, supporting the notion that this ionophore inhibits serotonin uptake by elevating [Na+]i (Figure 4: (top) Effects of increasing concentrations of monensin on ³H-serotonin uptake and [Na+]i in human platelets. Data are means + SD of six determinations). At about 70% inhibition of uptake, [Na+]i was elevated by about 20 mM (Figure 4 (bottom) Inhibition of specific ³H-serotonin uptake and increase of intracellular Na+ over baseline (Δ[Na+]i) in human platelets by monensin (500 nM)). This not only indicates similar properties for both cell systems, but also demonstrates for the first time the efficacy of hyperforin in a cell model expressing the human serotonin transporter. When investigated under similar conditions, hyperforin also increased [Na+]i in human platelets at the same concentrations needed to inhibit serotonin uptake (Figure 5:(top) Effects of increasing concentrations of hyperforin on ³H-serotonin uptake and [Na+]i in human platelets. Data are means + SD of six determinations, (bottom) Inhibition of specific ³H-serotonin uptake and increase of intracellular Na⁺ over baseline (Δ[Na+]i) in human platelets by hyperforin (5 µM)). Similar to monensin, the effect of hyperforin was maximal within 15 min incubation (Figure 6: Effects of increasing concentrations of citalopram on ³H-serotonin uptake and [Na⁺]ᵢ in human platelets. Data are means ± SD of six determinations). In contrast to monensin, hyperforin showed a U-shaped dose-response curve. Maximal elevation of [Na⁺]ᵢ by about 70 mM was seen at 50 µM hyperforin. Higher concentrations of hyperforin gave smaller effects (Fig. 6).

### Discussion

Our findings using mouse brain synaptosomes and human platelets demonstrate, that hyperforin, as many other antidepressant drugs, inhibits serotonin uptake in both systems with similar potencies. As far as we know, this is the first evidence that hyperforin is also active at the human serotonin transporter. However, the rather weak binding to the serotonin transporter labeled by ³H-paroxetine binding in comparision to the much stronger effect on serotonin uptake might indicate that the binding to the transporter site labeled by ³H-paroxetine is not the primary mechanism responsible for the uptake inhibition. The assumption is further supported by our observation of a non-competitive type of inhibition by hyperforin, contrary to the competitive type of inhibition seen for citalopram. Moreover, hyperforin is a rather non-selective synaptosomal uptake inhibitor (Müller et al., 1998; Chatterjee et al., 1998), which also argues against a specific binding site as primary mechanism of action.

Thus, we speculated that hyperforin works by elevating free intracellular sodium ([Na⁺]ᵢ). Since the sodium cotransport into the cell represents the driving force of the serotonin transporter, the serotonin uptake systems is very sensitive for conditions leading to reduced extracellular or enhanced intracellular sodium concentrations (Sneddon, 1996; Bogdanski, 1979).

An already known example is the sodium ionophore monensin, which elevates [Na⁺]ᵢ by promoting Na⁺/H⁺ exchange (Riddell et al., 1988; Katsuyoshi and Yoshihiko, 1991; Sandeaux et al., 1982). We could confirm previous findings that monensin dose-dependently inhibit ³H-serotonin uptake into brain synaptosomes (Reith and O'Reilly, 1990; Izenwasser et al., 1992). Inhibition into synaptosomes is non-competitive. Similar to hyperforin, monensin inhibits the synaptosomal uptake of norepinephrine, dopamine, GABA and L-glutamate with IC₅₀-values in the nanomolar range (unpublished observations). Thus, monensin effects on synaptosomal uptake systems show considerable similarities to the properties of hyperforin.

It was, however, not known to which extend the inhibition of serotonin uptake by monensin is associated with an increase of [Na⁺]ᵢ. Therefore, we compared the effects of increasing concentrations of monensin on both parameters in human platelets, which are well characterized cell systems for both types of experiments. As expected, monensin at concentrations sufficient to inhibit ³H-serotonin uptake led to a pronounced increase of [Na⁺]ᵢ. At an inhibition of about 75% (500 nM monensin) [Na⁺]ᵢ was elevated over baseline by about 25 mM [Na⁺]ᵢ. By contrast, citalopram at concentrations relevant for serotonin uptake inhibition had no effect at all on [Na⁺]ᵢ in human platelets. However, the effect of hyperforin was also associated with elevated [Na⁺]ᵢ. At a hyperforin concentration leading to about 75% uptake inhibition, again an elevation of [Na⁺]ᵢ by about 20 mM was found.

Our findings that hyperforin leads to an elevation of [Na⁺]ᵢ not only explains it's effects on serotonin uptake into platelets and synaptosomes, but also explains it's rather non-selective profile on many neurotransmitter transport systems, which all are driven by the Na⁺-gradient across the membrane (Letser et al., 1994). While hyperforin resembles in many ways the ionophore monensin, it shows a U-shaped dose-response curve and does not elevate [Na⁺]ᵢ up to the extracellular level as monensin does. Therefore, hyperforin is not simply a sodium ionophore, but its activity is associated with one of the major ion exchanging mechanism. This could explain that it's effects on [Na⁺]ᵢ get terminated once a certain level of [Na⁺]ᵢ is reached.

Hyperforin represents the first antidepressant drug known, which works by influencing [Na⁺]ᵢ.

### Abbreviations:

| | |
|---|---|
| GABA | γ-aminobutyric acid |
| HEPES | 4-(2-hydroxyethyl)-1-piperazineethansulfonic acid |
| PRP | Platelet rich plasma |
| SBFI/AM | 1,3-Benzenedicarboxylic acid, 4,4'-[1,4,10-trioxa-7,13-diazacyclopentadecan-7,13-diylbis(5-methoxy-6,2-benzofurandiyl)]bis-, tetraammonium salt |
| SSRI | Selective serotonin reuptake inhibitor |
| SD | Standard deviation |
| TCA | Tricyclic antidepressant |
| [Na⁺]ᵢ | Free intracellular sodium concentration |
| TRIS | Tris(hydroxymethyl)aminomethane |

### Claims

1. A method of treating humans to cure or prevent depression or other psychiatric disorders which comprisis treating human with an effective amount of a compound which is effective in elevating the intracellular concentration of Na⁺.
2. A method for screening for a pharmaceutical compound or composition for the curative or prophylactic treatment of depression or other psychiatric disorders comprising increasing the ability of a compound or a composition for elevation the intracellular concentration of Na⁺.
3. A method of using a compound or composition effective in elevating the intracellular concentration of Na⁺ for the manufacture of a medicament for the curative or prophylactic treatment of depression or other psychiatric disorders.
4. A method of any of the claims 1 to 3, whereas the compound or composition does not include hyperforin.
5. A method of any of the claims 1 to 4, whereas the free intracellular concentration of Na⁺ is increased by the compound or composition by 30 to 100 mM for minimum above the level of the untreated cell or to higher levels.
6. A method of any of the claims 1 to 5, whereas the compound or composition decreases the uptake of serotonin, norepinephrine, dopamine, GABA and/or L-glutamate or of any other neurotransmitter.
7. A method of any of the claims 1 to 6, whereas the compound or composition increases the uptake of Na⁺ in cells by Na⁺ -channels, Na⁺ -antiporters, Na⁺ -pumps or any other Na⁺ -exchange systems.
   - Celltypes in which intracellular Na+ concentration will be elevated:
   - Protozoen cells (plasmodium falciparum, plasmodium vinckei petteri and other protozoes)
   - Human and mammalian blood platelets
   - Human and mammalian synaptosomes
   - Human and mammalian erythrocytes
   - Human and mammalian lymphocytes
   - Human and mammalian neurons
   - Human and mammalian glial cells
8. A method of any of the claims 1 to 7, whereas the compound or composition interacts noncompetitive with the transporters of serotonin, norepinephrine, dopamine, GABA and/or L-glutamate or with any other neurotransmitter.
9. The use of hyperforin for the manufacture of a medicament for the curative or prophylactive treatment of interactions with protozoes.
10.A method of claim 2 whereas the ability of elevating (increasing) the intracellular concentration of Na⁺ is screened in synaptosomal preparation.
11. A method of claim 10 whereas the concentration of Na⁺ is measured with fluorescence spectroscopy.
12.A method of claim 11 whereas dyes for Na⁺ are used.
13.A method of claim 8 whereas the dose (of the compound or composition) - response (the intracellular concentration of Na+ )- curve shows a maximum.

### References

Bhattacharya SK, Chakrabarti A and Chatterjee, SS (1998) Active profiles of two hyperforin-containing hypericum extracts in behavioral models. *Pharmacopsychiatry* **31** Suppl. 1:22-29.

Biber A, Fischer H, Römer A and Chatterjee SS (1998) Oral bioavailability of hyperforin from hypericum extracts in rats and human volunteers. *Pharmacopsychiatry* **31** Suppl. 1:36-43.

Bogdanski DF, Tissari AH and Brodie BB (1979) Mechanism of transport and storage of biogenic amines. III. Effects of sodium and potassium on kinetics of 5-hydroxytryptamine and norepinephrine transport by rabbit synaptosomes. *Biochim Biophys Acta* **219**:189-199.

Borin M and Siffert W (1990) Stimulation by thrombin increases the cytosolic free Na⁺ concentration in human *platelets. J of Biol Chem* **265**:19543-19550.

Bradford MM (1976) A rapid and sensitive method for the quantitation of microgram quantities of protein utilizing the principle of protein-dye binding. *Anal Biochem* **72**:248-254.

Butterweck V, Wall A, Liefländer-Wulf U, Winterhoff H and Nahrstedt A (1998) Effects of the total extract and fractions of *hypericum perforatum* in animal assays for antidepressant activity. *Pharmacopsychiatry* **30** Suppl. 2:117-124.

Chatterjee SS, Bhattacharya SK, Wonnemann M, Singer A and Müller WE (1998) Hyperforin as a possible antidepressant component of hypericum extracts. *Life Sci* **63**:499-510.

Cott JM (1997) In vitro receptor binding and enzyme inhibition by *Hypericum perforatum* extract. *Pharmacopsychiatry* **30** Suppl. 2:108-112.

Dimpfel W, Schober M, Mannel M (1998) Effects of a methanolic extract and a hyperforin-enriched CO₂ extract of St. John's Wort (Hypericum perforatum) on intracerebral field potentials in the freely moving rat (Tele-Stereo-EEG). *Pharmacopsychiatry* **31** Suppl. 1:30-35.

Gurevich E and Joyce J (1996) Comparision of ³H-paroxetine and ³H-cyanoimipramine for quantitative measurement of serotonin transporter sites in human brain. *Neuropsychopharmacology* **14**:309-323.

Harootunian AT, Kao JPY, Eckert BK and Tsien RY (1989) Fluorescence ratio imaging of cytosolic free Na⁺ in individual fibroblasts and lymphocytes. *J Biol Chem.* **264**:19458-19467.

Hyttel J (1978) Effect of a specific 5-HT uptake inhibitor, citalopram (Lu 10-171), on ³H-5-HT uptake in rat brain synaptosomes in vitro. *Psychopharmacology* **60**:13-18.

Izenwasser S, Rosenberger JG and Cox BM (1992) Inhibition of [³H]dopamine and [³H]serotonin uptake by cocaine: Comparision between chopped tissue slices and Synaptosomes. *Life Sci.* **50**:541-547.

Kaehler ST, Sinner C, Chatterjee SS and Philippu A (1999) Hyperforin enhances the release of catecholamines, serotonin and glutamate in the rat locus coeruleus. *Neurosci Lett:* in press

Katsuyoshi Nakazato and Yoshihiko Hatano (1991) Monensin-mediated antiport of Na⁺ and H⁺ across liposome membrane. *Biochim Biophys Acta* **1064**:103-110

Laakmann G, Schüle C, Baghai T, Kieser M (1998) St. John's wort in mild to moderate depression: The relevance of hyperforin for the clinical efficacy. *Pharmacopsychiatry* **31** Suppl. 1:54-59.

Lester HA, Mager S, Quick MW and Corey JL (1994) Permeation properties of neurotransmitter transporters. *Annu Rev Pharmacol Toxicol* **34**:219-249.

Linde K, Ramirez G, Mulrow CD, Pauls A, Weidenhammer W and Melchart D (1996) St. John's Wort for depression - an overview and meta-analysis of randomised clinical trials. *Brit Med J* **313**:253-258.

Marcusson & Ross (1990) Binding of some antidepressants to the 5-HT transporter in brain and platelets. *Psychopharmacology* **102**:145.

Müller WE, Rolli M, Schäfer C and Hafner U (1997) Effects of hypericum extract (LI 160) in biochemical models of antidepressant activity. *Pharmacopsychiatry* **30** Suppl. 2:102-107.

Müller WE, Singer A, Wonnemann M, Hafner U, Rolli M and Schäfer C (1998) Hyperforin represents the neurotransmitter reuptake inhibiting constituent of hypericum extract. *Pharmacopsychiatry* **31** Suppl. 1:16-21.

Reith MEA and O'Reilly CA (1990) Inhibition of serotonin uptake into mouse brain synaptosomes by ionophores and ion-channel agents. *Brain Res* **521:**347-351.

Riddell FG, Arumugam S, Cox BG (1988) The monensin-mediated transport of Na⁺ and K⁺ through phospholipid bilayers studied by ²³Na- and ³⁹K-NMR. *Biochim Biophys Acta* **944:**279-284.

Sandeaux R, Sandeaux J, Gavach C and Brun B (1982) Transport of Na⁺ by monensin across bimolecular lipid membranes. *Biochim Biophys Acta* **684**:127-132.

Schellenberg R, Sauer S and Dimpfel W (1998) Pharmacodynamic effects of two different hypericum extracts in healthy volunteers measured by quantitative EEG. *Pharmacopsychiatry* **31** Suppl. 1:44-53.

Sneddon JM (1973) Blood platelets as a model for monoamine-containing neurones. *Prog Neurobiol* **1**:151-198.

Sneddon JM (1969) Sodium-dependent accumulation of 5-hydroxytryptamine by rat blood platelets. *Br J Pharmac* **37**:680-688.

Suzuki O, Katsumata Y, Oya M, Bladt S and Wagner H (1984) Inhibition of monoamine oxidase by hypericin. *Planta Med* **50**:272-274.

Volz HP (1997) Controlled clinical trials of hypericum extracts in depressed patients - an overview. *Pharmacopsychiatry* **30** Suppl. 2:72-76.

Wheatley D (1998) Hypericum extract - Potential in the treatment of depression. *CNS Drugs* **9**: 431-440.

Wong AHC, Smith M, Boon HS (1998) Herbal remedies in psychiatric practice. *Arch Gen Psychiatry* **55**:1033-1044.

## Claims

1. A method of treating humans to cure or prevent depression or other psychiatric disorders which comprisis treating human with an effective amount of a compound which is effective in elevating the intracellular concentration of Na⁺.

2. A method for screening for a pharmaceutical compound or composition for the curative or prophylactic treatment of depression or other psychiatric disorders comprising increasing the ability of a compound or a composition for elevation the intracellular concentration of Na⁺.

3. A method of using a compound or composition effective in elevating the intracellular concentration of Na⁺ for the manufacture of a medicament for the curative or prophylactic treatment of depression or other psychiatric disorders.

4. A method of any of the claims 1 to 3, whereas the compound or composition does not include hyperforin.

5. A method of any of the claims 1 to 4, whereas the free intracellular concentration of Na⁺ is increased by the compound or composition by 30 to 100 mM for minimum above the level of the untreated cell or to higher levels.

6. A method of any of the claims 1 to 5, whereas the compound or composition decreases the uptake of serotonin, norepinephrine, dopamine, GABA and/or L-glutamate or of any other neurotransmitter.

7. A method of any of the claims 1 to 6, whereas the compound or composition increases the uptake of Na⁺ in cells by Na⁺ -channels, Na⁺ -antiporters, Na⁺ -pumps or any other Na⁺ -exchange systems.
- Celltypes in which intracellular Na+ concentration will be elevated:
- Protozoen cells (plasmodium falciparum, plasmodium vinckei petteri and other protozoes)
- Human and mammalian blood platelets
- Human and mammalian synaptosomes
- Human and mammalian erythrocytes
- Human and mammalian lymphocytes
- Human and mammalian neurons
- Human and mammalian glial cells

8. A method of any of the claims 1 to 7, whereas the compound or composition interacts noncompetitive with the transporters of serotonin, norepinephrine, dopamine, GABA and/or L-glutamate or with any other neurotransmitter.

9. The use of hyperforin for the manufacture of a medicament for the curative or prophylactive treatment of interactions with protozoes.

10. A method of claim 2 whereas the ability of elevating (increasing) the intracellular concentration of Na⁺ is screened in synaptosomal preparation.

11. A method of claim 10 whereas the concentration of Na⁺ is measured with fluorescence spectroscopy.

12. A method of claim 11 whereas dyes for Na⁺ are used.

13. A method of claim 8 whereas the dose (of the compound or composition) response (the intracellular concentration of Na⁺)- curve shows a maximum.
